# EUROPÄISCHE PATENTANMELDUNG

(11) **EP 3 088 018 A1**
(43) Veröffentlichungstag der Anmeldung: **02.11.2016**
(21) Anmeldenummer: 15166045.3
(22) Anmeldetag: 30.04.2015
(51) Int. Cl.: A61M 1/10, A61M 1/12

(54) **ROTOR FÜR EINE FLUIDPUMPE SOWIE VERFAHREN UND GIESSFORM FÜR SEINE HERSTELLUNG**

(71) Anmelder: ECP Entwicklungsgesellschaft mbH, 12247 Berlin (DE)
(72) Erfinder: Sieß, Thorsten, 52074 Aachen (DE)
(74) Vertreter: Pfenning, Meinig & Partner mbB

(57) **Zusammenfassung**

Gezeigt wird ein Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem komprimierten Zustand und einem expandierten Zustand komprimierbar und expandierbar ist und der wenigstens teilweise aus einem durch Fasern (10, 11,13, 18, 19) verstärkten Kunststoff besteht und zur Rotation um eine Rotationsachse (14) vorgesehen ist, dadurch gekennzeichnet, dass ein erster Anteil von mehr als 30%, insbesondere mehr als 50%, der Fasern (10, 11,13,18,19) im expandierten Zustand des Rotors (42) im Wesentlichen gestreckt von ihrem jeweils der Rotationachse am nächsten liegenden ersten Ende (10a, 11a, 13a) zu einem der Rotationsachse ferner liegenden zweiten Ende (10b, 11b, 13b) verläuft. Der Rotor bietet gemäß der Erfindung eine hohe Formtreue auch bei wiederkehrender mechanischer Belastung.

## Beschreibung

Die vorliegende Schutzrechtsanmeldung liegt auf dem Gebiet der Mechanik und befasst sich konkret mit Rotoren für Fluidpumpen. Sie ist mit besonderem Vorteil im Bereich der Medizintechnik bei Katheterpumpen einsetzbar.

Im Bereich der Fluidpumpen sind Rotorpumpen bereits in verschiedenen Ausführungsformen als Axialpumpen oder Radialpumpen bekannt. In beiden Fällen wird das zu fördernde Fluid durch eine Rotation eines Rotors und an diesem befestigter Förderelemente beschleunigt, entweder in Axialrichtung oder in Radialrichtung.

Derartige Pumpen können grundsätzlich auch nach dem Stand der Technik bereits komprimiert werden, um sie platzsparend anzuordnen oder zu transportieren. Dies trifft insbesondere auf Katheterpumpen für den medizinischen Anwendungsbereich zu, die oft radial komprimierbar und expandierbar sind, um sie durch einen Katheter bzw. durch Hohlräume im Körper eines Patienten an einen Anwendungsort befördern und dort expandieren zu können, bevor die Inbetriebnahme erfolgt. Solche Pumpen werden beispielsweise zur Unterstützung eines Herzens eines Patienten bei der Blutförderung eingesetzt und zu diesem Zweck durch ein Blutgefäß bis an oder in eine Herzkammer vorgeschoben.

Dabei ergeben sich besondere Herausforderungen durch die geringe Größe des Rotors und zusätzlich seine Komprimierbarkeit. Im expandierten Zustand muss der Rotor trotz seiner Komprimierbarkeit reproduzierbar eine Betriebsform einnehmen, die sich auch im Betrieb bei möglichst hohen Förderdrehzahlen so wenig wie möglich ändert, um eine Verringerung der Effizienz sowie eine Schädigung der zu fördernden Blutbestandteile zu verhindern.

Aus diesem Grund ist bereits der Einsatz verschiedenster Materialien und Materialkombinationen zu dem genannten Zweck erwogen und untersucht worden. Beispielsweise ist aus der WO 2010/063494 A1 der Einsatz verschiedenster Elastomere, auch im Zusammenhang mit einer Faserverstärkung, bereits bekannt.

Aus der WO 2012/007141 A1 ist eine Verstärkung eines Pumpenrotors durch Fasern bekannt, die in orientierter Form, beispielsweise in Radialrichtung, im Rotor angeordnet sein können.

Schließlich ist aus der WO 2012/007140 A1 ein Pumpenrotor mit Verstärkungselementen bekannt, die im Wesentlichen außerhalb der Förderelemente, beispielsweise auf deren Oberflächen, vorgesehen sein können.

Vor dem Hintergrund des Standes der Technik liegt der vorliegenden Erfindung somit die Aufgabe zugrunde, einen Kunststoffrotor der oben genannten Art zu schaffen, der eine minimale Relaxation nach Verformungen zwischen dem komprimierten und dem expandierten Zustand sowie eine möglichst genaue Reproduzierbarkeit seiner Geometrie zumindest im expandierten Zustand aufweist.

Die Aufgabe wird durch einen Rotor, ein Verfahren zur Herstellung eines Rotors sowie eine entsprechende Gießform für einen Rotor gelöst.

Es ergibt sich unter anderem dadurch ein Rotor für eine komprimierbare Fluidpumpe, insbesondere eine durch ein Blutgefäß in einen Patientenkörper einführbare Blutpumpe, der ein oder mehrere Förderelemente aufweist und radial zwischen einem komprimierten Zustand und einem expandierten Zustand komprimierbar und expandierbar ist und der wenigstens teilweise aus einem durch Fasern verstärkten Kunststoff besteht und zur Rotation um eine Rotationsachse vorgesehen ist, wobei der Kunststoff eine Shorehärte < 100 D aufweist.

Durch die geringe Shore-Härte, die beispielsweise auch < 80 D gewählt werden kann, ergibt sich bei starker Biegung oder Knickung des Materials für die eingebetteten Fasern die Möglichkeit, in das nachgiebige Material der Basis auszuweichen und dadurch den Krümmungsradius nach unten zu begrenzen. Damit ergibt sich eine Verringerung der Bruchgefahr für die Fasern.

Zudem kann bei einem solchen Rotor auch vorgesehen sein, dass ein erster Anteil der Fasern im expandierten Zustand des Rotors im Wesentlichen gestreckt von ihrem jeweils der Rotationsachse am nächsten liegenden ersten Ende zu einem der Rotationsachse ferner liegenden zweiten Ende verläuft. Durch diese Positionierung, Ausrichtung und Formung der Fasern wird eine Überstreckung des Rotors über den expandierten Zustand hinaus wirkungsvoll vermieden.

Zudem ergibt sich ein Rotor für eine komprimierbare Fluidpumpe, insbesondere eine durch ein Blutgefäß in einen Patientenkörper einführbare Blutpumpe, der ein oder mehrere Förderelemente aufweist und radial zwischen einem komprimierten Zustand und einem expandierten Zustand komprimierbar und expandierbar ist und der wenigstens teilweise aus einem durch Fasern verstärkten Kunststoff besteht und zur Rotation um eine Rotationsachse vorgesehen ist, wobei ein erster Anteil von mehr als 30%, insbesondere mehr als 50%, der Fasern im expandierten Zustand des Rotors im Wesentlichen gestreckt von ihrem jeweils der Rotationachse am nächsten liegenden ersten Ende zu einem der Rotationsachse ferner liegenden zweiten Ende verläuft.

Durch eine derartige Anordnung und Ausrichtung eines ausreichenden Anteils der Fasern in dem Rotor, insbesondere innerhalb der Förderelemente des Rotors, wird eine optimierte Stabilisierung des Rotors im expandierten Zustand erreicht. Eine weitere Verformung wird durch die Verstärkungsfasern praktisch verhindert, oder es wird zumindest sichergestellt, dass nach einer Überstreckung eine Rückkehr in die vorherige Rotorgeometrie erfolgt. Auch gewisse Veränderungen, wie beispielsweise Materialkriechen des Kunststoffs, aus dem der Rotor besteht, können somit keine dauerhafte Geometrieänderung des Rotors im expandierten Zustand hervorrufen. Dies ist beispielsweise bei Verwendung eines Polymerrotors aus einem Thermoplasten oder aus einem thermoplastischen oder einem chemisch schwach kreuzvernetzten Elastomer zur Herstellung des Rotors erreichbar.

Als Material für die Fasern kommt dabei insbesondere Glas, jedoch auch Kohlenstoff bzw. Polycarbonat in Betracht. Die Fasern werden hierzu während des Herstellungsprozesses, beispielsweise eines Spritzgussverfahrens oder eines Vakuumvergussverfahrens, in das Polymer eingebracht. Die einzelnen Fasern weisen üblicherweise einen hohen E-Modul auf und werden vorteilhaft in einem gestreckten, nicht gebogenen Zustand in die Matrix integriert. Zur Stabilisierung des Rotors muss ein ausreichender Anteil der Fasern der erfindungsgemäßen Bedingung genügen, beispielsweise mehr als 30% der insgesamt in dem Rotormaterial befindlichen Fasern, gemessen an der Fasermasse oder am Faservolumen.

Vorteilhaft können wenigstens 50% der vorhandenen Fasern gemäß der Erfindung entsprechend eingebracht, positioniert und ausgerichtet sein. Es kann auch vorgesehen sein, mehr als 50%, beispielsweise 60% oder 70% oder sogar 80% oder 90% der Fasern entsprechend zu positionieren und auszurichten. Dabei kann ein möglichst großer Anteil der Fasern vorteilhaft in einer biegeneutralen Ebene innerhalb des Volumens des jeweiligen Förderelements des Rotors angeordnet sein, um keine Längsstauchung oder Dehnung der Fasern bei der Komprimierung oder Expandierung hervorzurufen. Es kann jedoch auch gezielt eine Positionierung parallel im Abstand zu der biegeneutralen Ebene angestrebt werden, um eine besondere Stabilisierung bei Ausbiegung der Förderelemente in eine Richtung durch Streckung der Fasern zu erreichen. Jedenfalls sollten die Fasern vorteilhaft im Inneren der Förderelemente verlaufen und einen bestimmten Mindestabstand zu den äußeren Begrenzungsflächen der Förderelemente einhalten.

Es können in dem Rotor auch mehrere Gruppen von Fasern unterschiedlicher Länge vorgesehen sein, wobei wenigstens eine Gruppe eine bestimmte Mindestlänge aufweist, während die Fasern von einer oder mehreren Gruppen kürzer sind oder eine Längenverteilung aufweisen, die nur vernachlässigbar wenige Fasern oberhalb einer Faserlänge aufweist, die unterhalb einer typischen Faserlänge des ersten Anteils der Fasern liegt. Typisch liegt die Durchschnittslänge der kürzeren Fasern bei weniger als einem Drittel der Länge des ersten Anteils der Fasern.

Der Rotor kann vorteilhaft dadurch ausgestaltet werden, dass jede Faser des ersten Anteils der Fasern in ihrem Verlauf höchstens 45° in Axialrichtung und/oder Azimutalrichtung von einer radial auf die Rotorachse (14) ausgerichteten Lage abweicht. Die Fasern verlaufen dann in einer Fläche, in der auch die gesamte Rotationsachse des Rotors verläuft, so dass die Fasern sich beispielsweise direkt von der Rotationsachse senkrecht radial nach außen erstrecken können. Jedoch ist auch eine Ausrichtung der Fasern von der Rotationsachse radial in einem Winkel zwischen 45° und 90° zur Rotationsachse möglich. In einer Ausgestaltung weist die Erstreckung der Fasern jedenfalls keine oder nur geringe azimutale (in Umfangsrichtung verlaufende) Ausrichtung auf.

Eine weitere Ausgestaltung sieht vor, dass jede Faser des ersten Anteils der Fasern im Wesentlichen senkrecht zur Rotationsachse verläuft. Hiermit ergibt sich eine besonders effiziente Stabilisierung der Förderelemente, wenn diese in Umfangsrichtung in Bezug auf die Achse des Rotors gebogen und beispielsweise zur Komprimierung an eine Rotornabe angelegt werden.

Es kann auch einfach ein radialer Verlauf der Fasern in Bezug auf die Rotationsachse verwirklicht werden.

Eine weitere Ausgestaltung sieht vor, dass jede Faser des ersten Anteils der Fasern entlang der Längsachse eines Förderelements verläuft. Durch eine derartige Anordnung der Fasern werden die einzelnen Förderelemente besonders effizient in ihrer expandierten Form stabilisiert. Sinnvoll kann dabei sein, dass die Fasern sich über den Bereich radial erstrecken, in dem die größte Verformung bei der Komprimierung und Expandierung des Rotors erfolgt. Es kann jedoch auch gerade vorgesehen sein, die Fasern derart anzuordnen, dass der Bereich, in dem die stärkste Verformung bei der Komprimierung und Expandierung des Rotors erfolgt, frei von Verstärkungsfasern gelassen oder nur mit einem verringerten Anteil von Verstärkungsfasern ausgestattet wird.

Unter der Längsachse eines Förderelements wird im Wesentlichen die Erstreckungsrichtung des Förderelements radial in Bezug auf die Rotorachse des Rotors verstanden. Dies trifft auch dann zu, wenn die Höhe des jeweiligen Förderelements in Axialrichtung des Rotors größer ist als seine radiale Erstreckung.

Um eine ausreichende Stabilisierung des Rotors bzw. der Förderelemente im expandierten Zustand zu erreichen, kann vorteilhaft vorgesehen sein, dass die Länge der Fasern des ersten Anteils der Fasern wenigstens 10%, insbesondere wenigstens 30%, noch vorteilhafter wenigstens 50%, des Radius des Rotors beträgt. Dabei ist es besonders vorteilhaft, wenn der erste Anteil der Fasern, die eine derart beschaffene Faserlänge aufweisen, wenigstens 70% der insgesamt vorhandenen Fasern darstellt, gemessen anhand der Zahl der Fasern oder anhand der Masse der Fasern.

Um ein Brechen der Fasern bei maximaler Komprimierung des Rotors zu verhindern, sollte ein bestimmter Maximaldurchmesser der einzelnen Fasern insbesondere bei der Herstellung aus Glas nicht überschritten werden. Deshalb kann vorteilhaft zur Ausgestaltung der Erfindung vorgesehen, dass der Durchmesser der Fasern des ersten Anteils der Fasern kleiner als 40 µm ist. Diese Durchmesserbedingung sollte möglichst für alle Fasern des ersten Anteils der Fasern, jedoch mindestens für 90% oder 80% des ersten Anteils der Fasern gelten, sofern bei der Herstellung eine gewissen Streuung der Durchmesserwerte nicht zu vermeiden ist.

Um bei einer maximalen Verformung des Rotors, beispielsweise beim Knicken bestimmter Stellen der Förderelemente, einen Bruch der Fasern zu verhindern, kann in einer weiteren vorteilhaften Ausgestaltung beispielsweise vorgesehen sein, dass der Kunststoff, in den die Fasern eingebettet sind, eine Shorehärte < 100 D aufweist. Bei einer derartigen Shorehärte bzw. Nachgiebigkeit des Materials ist jeweils gewährleistet, dass bei einer starken Verformung die Fasern in dem Material der Matrix ausreichend ausweichen können, um einen bestimmten Biegeradius nicht zu unterschreiten. Damit werden die Fasern des ersten Anteils von Fasern vor Bruch geschützt.

Der Gegenstand kann weiterhin dadurch ausgestaltet sein, dass die Förderelemente aus einem Schaumstoff bestehen. Dabei ist insbesondere an einen geschlossenporigen Schaumstoff gedacht, der durch die Verstärkungsfasern effektiv stabilisierbar ist und der dennoch einfach und in ausreichendem Maß komprimierbar ist. Bei der Kompressionsbewegung ist gerade bei einem Schaumstoff ein Ausweichen der Fasern zur Verhinderung der Unterschreitung eines kritischen Biegeradius besonders einfach möglich. Üblicherweise weisen derartige Schaumstoffe im Inneren des Volumens der Förderelemente entsprechende Poren auf, sind jedoch an den äußeren Begrenzungsflächen praktisch vollständig geschlossen.

Die Neuerung bezieht sich außer auf einen Rotor der oben beschriebenen Art auch auf ein Verfahren zur Herstellung eines Rotors mittels eines Gießverfahrens , insbesondere eines Spritzgießverfahrens, bei dem das Material der Förderelemente in Radialrichtung in Bezug auf die Rotorachse in die Volumina der Förderelemente derart eingebracht wird, dass der Spritzgusswerkstoff in die Volumina der Förderelemente jeweils in Radialrichtung hineinströmt.

Durch das weit überwiegend radiale Einströmen des Spritzgusswerkstoffs in die Gießform bzw. in die Volumina der Förderelemente werden auch die Verstärkungsfasern in der Einströmrichtung, d. h. in radialer Richtung, mitgenommen und eingebettet. Hierdurch lässt sich die beanspruchte Positionierung und Orientierung der Fasern im Material des Rotors in besonders einfacher und effektiver Weise gewährleisten.

Es kann auch vorgesehen sein, dass der Spritzgusswerkstoff in die Volumina der Förderelemente jeweils von dem der Rotorachse nächsten Bereich aus oder von dem der Rotorachse fernsten Bereich aus in radialer Richtung eingespritzt wird.

Die Neuerung bezieht sich zudem auf eine Gießform für einen Rotor der oben beschriebenen Art, bei der vorteilhaft vorgesehen ist, dass bei den Volumina der Förderelemente an deren radial verlaufenden Kanten Überströmkanäle vorgesehen sind, um einen störungsfreien Fluss des Gießwerkstoffs in Radialrichtung zu ermöglichen. Durch das Vorsehen von Überströmkanälen an den Kanten der Förderelemente ist sichergestellt, dass nicht durch Verwirbelungen des einströmenden Spritzgusswerkstoffs an den Wänden der Gussform in diesem Bereich die Fasern mitgenommen und so verformt werden, dass sie den Wirbeln folgen und nicht mehr in gestreckt radialer Orientierung in dem Rotor vorliegen. Es ist ferner vorgesehen, das Polymer, aber nicht die Fasern (die Höhe der Überströmkanäle ist folglich in wenigstens einer Ausdehnungsrichtung kleiner als der Faserdurchmesser) in Teilen abführen zu können. Hierdurch kann es zu einer erwünschten Aufkonzentration der Fasern in den Förderelementen kommen. Ferner wird durch das gezielte Überströmen die radiale Ausrichtung der Fasern verbessert. Der entsprechend überströmende Spritzgusswerkstoff kann später nach dem Erhärten von den Förderelementen entfernt werden. Unter den Längskanten der Förderelemente werden die in expandierter Form im Wesentlichen in Radialrichtung verlaufenden Kanten der Förderelemente verstanden, die bei der Wechselwirkung der Förderelemente mit dem zu fördernden Fluid im Wesentlichen die vorlaufende und nachlaufende Kante des jeweiligen Förderelements, beispielsweise einer Förderschaufel, bilden.

Im Folgenden wird die Neuerung anhand von Ausführungsbeispielen in Figuren einer Zeichnung gezeigt und nachfolgend erläutert. Dabei zeigt
- Fig. 1: schematisch in einem Schnitt eine Katheterpumpe, die in eine Herzkammer eines Patienten eingeführt ist,
- Fig. 2a: einen Ausschnitt aus einem Rotor einer Katheterpumpe zur Förderung von Blut,
- Fig. 2b: eine Querschnittsdarstellung eines entspannten Rotors mit einer beispielhaft dargestellten Faser,
- Fig. 3: eine schematische Darstellung einer Ebene, die die Rotationsachse des Rotors enthält,
- Fig. 4: einen Ausschnitt aus einem planen Förderelement mit einer Faser zur Verstärkung,
- Fig. 5a: die Ausrichtung einer Faser beim Gießvorgang,
- Fig. 5b: eine Seitenansicht des Abschnitts aus Figur 4,
- Fig. 6: den Abschnitt eines Förderelements wie in Figur 4 und 5 gezeigt nach einem Knickvorgang,
- Fig. 7: eine schematische Ansicht eines Gießwerkzeugs für einen Rotor, wobei der Gießvorgang durch Strömungspfeile angedeutet ist,
- Fig. 8: eine Gießform für einen Rotor mit einer verglichen mit Figur 7 umgekehrten Einspritzrichtung sowie
- Fig. 9: eine Gießform, bei der Maßnahmen gegen eine Verwirbelung des Spritzgusswerkstoffs im Volumen der Förderelemente getroffen sind.

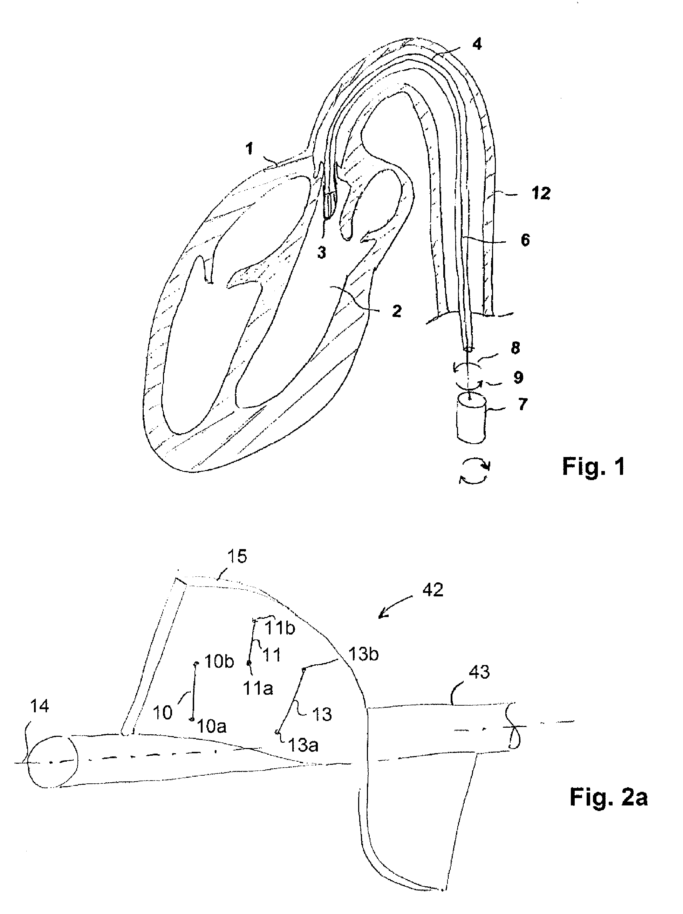

Figur 1 zeigt im Querschnitt das Herz 1 eines Patienten mit mehreren Herzkammern, wobei die Herzkammer 2 mit der Aorta 12 in Verbindung steht. Durch die Aorta 12 ist ein Katheter 4 bis in die Herzkammer 2 vorgeschoben, wobei am Ende des Katheters 4 ein Pumpenkopf 3 mit einer Rotationspumpe angeordnet ist. Die Rotationspumpe ist mittels einer durch den Katheter 4 verlaufenden drehbaren Welle 6 antreibbar, die innerhalb des Pumpenkopfes 3 mit einem Pumpenrotor 42 verbunden ist. Der Pumpenrotor rotiert in einem nicht im Einzelnen dargestellten Gehäuse des Pumpenkopfes 3.

Die biegsame Welle 6 ist mit einem Motor 7 verbunden, der beispielsweise außerhalb des Patientenkörpers angeordnet ist. Das Drehmoment kann beispielsweise über eine Magnetkopplung von dem Motor 7 in beiden Drehrichtungen 8, 9 auf die Welle 6 übertragen werden.

Der Katheter 4 wird üblicherweise über eine Schleuse vom Körperäußeren durch die Haut und Gewebe sowie die Gefäßwand in die Aorta 12 hinein und in dieser vorgeschoben.

Die Pumpe saugt in der Herzkammer 2 Blut an und fördert dies in die Aorta 12. Hierdurch kann die Herzpumpe die Funktion des Herzens 1 entweder unterstützen oder zumindest zeitweise ersetzen.

Neben der in der Figur dargestellten Katheterpumpe mit mechanischem Antrieb sind auch andere Pumpen, insbesondere zur intrakorporalen Anwendung, Gegenstand dieses Schutzrechts, beispielsweise Pumpen mit hydraulischem oder elektrischem Antrieb, und dort auch Pumpen, bei denen der Antrieb innerhalb des menschlichen Körpers ist.

Die Pumpe wird mitsamt dem Pumpenkopf, dem Pumpengehäuse und dem Rotor zur Verschiebung in der Aorta radial komprimiert und beispielsweise innerhalb des Katheters 4 verschoben. Die Pumpe kann dann aus dem Katheter 4 axial herausgeschoben werden und sich radial entfalten, d. h. expandiert werden. Dabei werden hohe Anforderungen an die Materialien des Pumpengehäuses und insbesondere des Pumpenrotors gestellt: Die Förderelemente des Pumpenrotors weisen eine sehr geringe Wandstärke auf, müssen aber dennoch auch bei hohen Rotationdrehzahlen formstabil bleiben und in reproduzierbarer Weise Blut fördern.

Zu diesem Zweck sind in die Matrix des Kunststoffs, aus dem der Rotor besteht, Verstärkungsfasern (Fasern) eingebettet, die beispielsweise als Glasfasern oder Polycarbonatfasern ausgestaltet sein können. Solche Fasern sind in Figur 2a in drei Einzelbeispielen dargestellt. Es sind die drei Fasern 10, 11, 13 gezeigt, von denen jede ein erstes Ende oder einen ersten Abschnitt 10a, 11a, 13a aufweist, das/der der Rotationsachse 14 näher ist als das jeweilige zweite Ende / der jeweilige zweite Abschnitt 10b, 11b, 13b der Fasern 10, 11, 13.

Die Fasern 10, 11, 13 erstrecken sich im Wesentlichen von der Rotationsachse oder einem Punkt in der Nähe der Rotationsachse 14 des Rotors radial nach außen weg. Dabei ist es nicht notwendig, dass der Rotor 42, wie im dargestellten Beispiel, eine Nabe 43 aufweist. Das wendelartige Förderelement 15 kann auch eine derartige Eigenstabilität aufweisen, dass eine Rotornabe nicht notwendig ist.

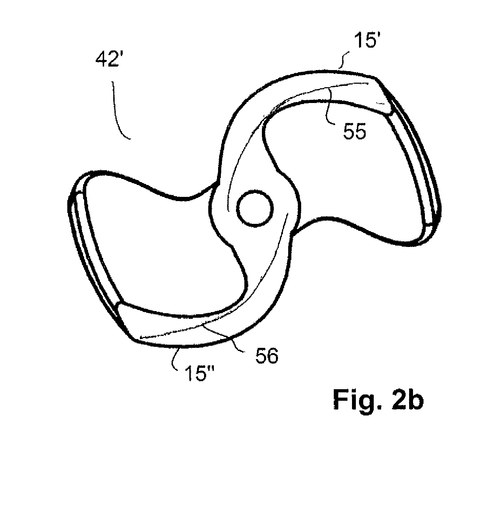

Grundsätzlich kann die Kunststoffmatrix des Förderelements oder der Förderelemente 15 mit Fasern verstärkt sein, die in Bezug auf Länge und/oder Dicke und/oder Orientierung ungleichmäßig verteilt und angeordnet sind. Ein Aspekt ist, dass ein gewisser Mindestanteil der Fasern im expandierten Zustand des Rotors, der in Figur 2 dargestellt ist, im Wesentlichen gestreckt von der Rotationsachse weg verläuft. Der Anteil der Fasern, der die genannte Bedingung erfüllt, von der Gesamtmenge der Fasern, die in den Kunststoff des Rotors eingebettet sind, sollte wenigstens 30% oder vorteilhaft 50% oder noch vorteilhafter beispielsweise 70%, gemessen in Volumen- oder Massenprozent der Fasern oder auch in der Zahl der Fasern, darstellen. Dabei ist vorteilhaft eine gewisse Mindestlänge der Fasern gegeben, beispielsweise etwa wenigstens 20% oder wenigstens 40% oder 50% des Radius des Rotors. Die Fasern können während des Füllvorgangs der Form leicht aus der axialen Ausrichtung, die der Nabe 43 entspricht, in die radiale Position der Förderelemente 15 wechseln ohne hierfür gebogen zu werden, da der Winkel zwischen dem Förderelement 15 und der Nabe 43 mit < 30° oder bevorzugt < 20° relativ flach verläuft. Somit werden die Fasern bei der radialen Befüllung der Förderelemente 15 einfach von der sie umgebenden Matrix mitgenommen und mit dem Materialfluss im Förderelement radial gemäß Figur 2 ausgerichtet.

Weitere vorteilhafte Eigenschaften der Fasern sind eine gewisse Maximaldicke, wobei ein Durchmesser von höchstens 40 µm vorteilhaft sein kann, um bei einer starken Biegung der Fasern keinen Bruch der Fasern selbst zu erzeugen. Mit ca. 40 µm hingegen sind die Fasern biegesteif genug, um die sie umgebende Matrix nach erfolgter Deformation wieder in den Ausganszustand zurück zu holen und ein nachhaltiges Kriechen der Matrix unter dauerhafter Biegebelastung zu verhindern. Ferner sind Fasern mit 40 µm Durchmesser druck- und zugsteif, so dass sie bei einer Anordnung außerhalb des biegeneutralen Bereiches ebenfalls ein rückstellendes Moment erzeugen und einer bleibenden Deformation entgegen wirken.

Die Fasern können für eine Verbesserung der Verbindung mit der Matrix mit einem Haftvermittler beschichtet sein.

Figur 2b zeigt im Querschnitt einen Rotor 42' mit Förderelementen 15', 15" im entspannten, expandierten Zustand. Die Fasern 55, 56 weisen in diesem Zustand eine möglichst gestreckte Form auf, so dass sie einer weitergehenden Verformung des Rotors durch ihre Längssteifigkeit einen Widerstand entgegensetzen.

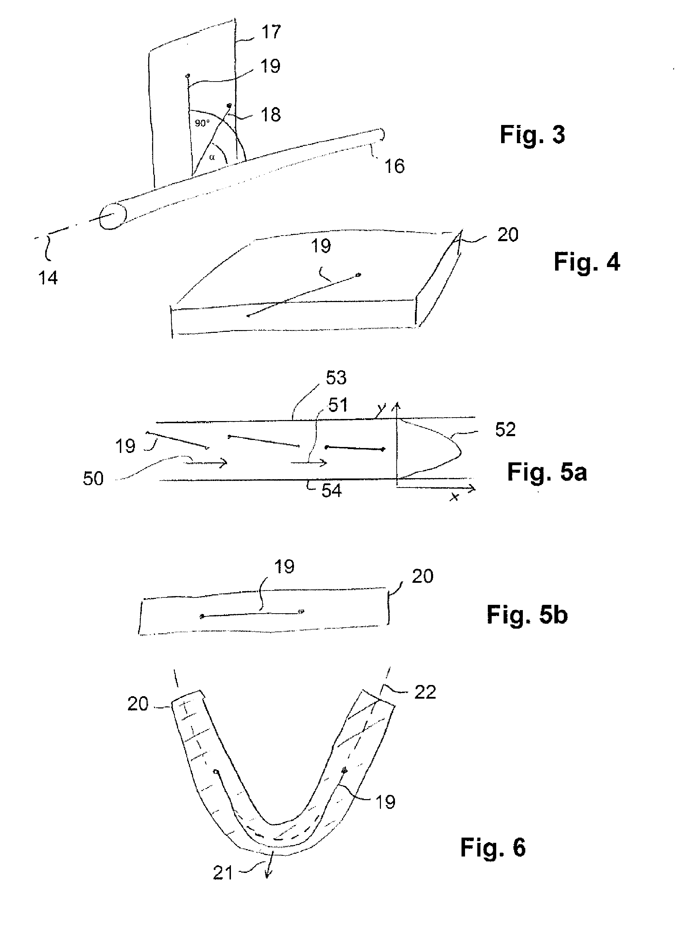

Anhand der Figur 3 soll näher die mögliche Orientierung der Fasern erläutert werden. Die Rotornabe ist in Figur 3 mit 16 bezeichnet, und die Rotationsachse mit 14. Es ist eine Ebene 17 anhand eines ausgeschnittenen Rechtecks dargestellt, wobei die Ebene 17 die Rotationsachse 14 enthält, d. h., die Rotationsachse 14 verläuft vollständig in der Ebene 17.

Es sind beispielhaft zwei Fasern 18, 19 eingezeichnet, die beide in der Ebene 17 im Wesentlichen radial in Bezug auf die Rotationsachse 14 verlaufen. Die Faser 18 verläuft in einem Winkel α zur Rotationsachse 14, teilweise in Axialrichtung, wobei der Winkel α vorteilhaft zwischen 45° und 90° liegt. Die Faser 19 ist derart ausgerichtet, dass sie senkrecht auf der Rotationsachse 14 steht. Ein reales Schaufelblatt ist in drei Dimensionen wendelförmig gebogen, so dass in vielen Fällen eine begrenzte Erstreckung der Fasern in Azimutalrichtung hinzukommt.

Die einzelnen Fasern müssen nicht derart positioniert sein, dass sie im Bereich der Rotationsachse 14 oder der Rotornabe 16 ihren ersten Anfangspunkt/ Endpunkt haben. Sie können auch so angeordnet sein, dass sie zwischen zwei Endpunkten verlaufen, die beide von der Rotorachse 14 und/oder von der Rotornabe 16 radial beabstandet sind. Sie können sich aber auch von einem ersten radial, außen liegenden Schaufelrand bis zu einem zweiten, radial gegenüber liegenden Schaufelrand, jedenfalls über die Achse hinweg, erstrecken.

In Figur 4 ist schematisch ein Ausschnitt 20 aus einem Förderelement dargestellt, wobei der Ausschnitt 20 quaderförmig gestaltet ist. In dem Abschnitt 20 ist eine Faser 19 dargestellt.

In Figur 5a wird deutlich gemacht, wie die zentrale Ausrichtung der Faser 19 erreicht wird. Das bevorzugt laminare Strömungsprofil beim Befüllen der Gießform zwischen den Begrenzungswänden 53 und 54 hat die höchste Strömungsgeschwindigkeit in der Mitte und die niedrigste Geschwindigkeit in der Nähe der Bewandung. Die nun zunächst windschief liegende Faser 19 ist in drei von links nach rechts nacheinander erreichten Winkelpositionen dargestellt und wird durch die Materialströmung, angedeutet durch die Pfeile 50, 51, infolge der Geschwindigkeitsverteilung in der Gießform in die Mitte des Strömungsprofils gezogen. Die Geschwindigkeitsverteilung der Strömung ist in dem Graphen 52 des Diagramms mit den Achsen x (=̂ Geschwindigkeit) und y (=̂ Ortskoordinate in der Gießform) dargestellt.

Figur 5b zeigt eine Seitenansicht, wobei das Material des Abschnitts 20 transparent dargestellt ist, so dass der Verlauf der Faser 19 etwa in der Mitte zwischen den Begrenzungsflächen des Förderelements sichtbar ist.

Nun soll anhand der Figur 6 das Verhalten der Faser bei einer starken Biegung oder Knickung des Förderelements beschrieben werden.

Figur 6 zeigt den Abschnitt 20 des Förderelements nach einer Knickung. Die Faser 19 ist ebenfalls verformt. Durch eine gewisse Eigensteifigkeit der Faser kann diese jedoch wegen der Nachgiebigkeit des Materials des Förderelements beim Knicken nach außen in Richtung des Pfeils 21 ausweichen, um einen möglichst großen Krümmungsradius der Faser zu erreichen und damit einem Bruch der Faser entgegenzuwirken. Im Bereich der Knickstelle ist damit die Faser 19 aus der Mitte zwischen den Begrenzungswänden des Förderelements für die Zeitdauer der Knickung ausgelenkt. In Figur 6 ist die Mittelebene des Förderelements der Übersichtlichkeit halber wenigstens abschnittsweise durch eine gestrichelte Linie 22 dargestellt. Um diesen Effekt zu erreichen, ist eine Weichheit der Kunststoffmatrix des Rotors vorteilhaft, die einer Shorehärte < 100 D entspricht.

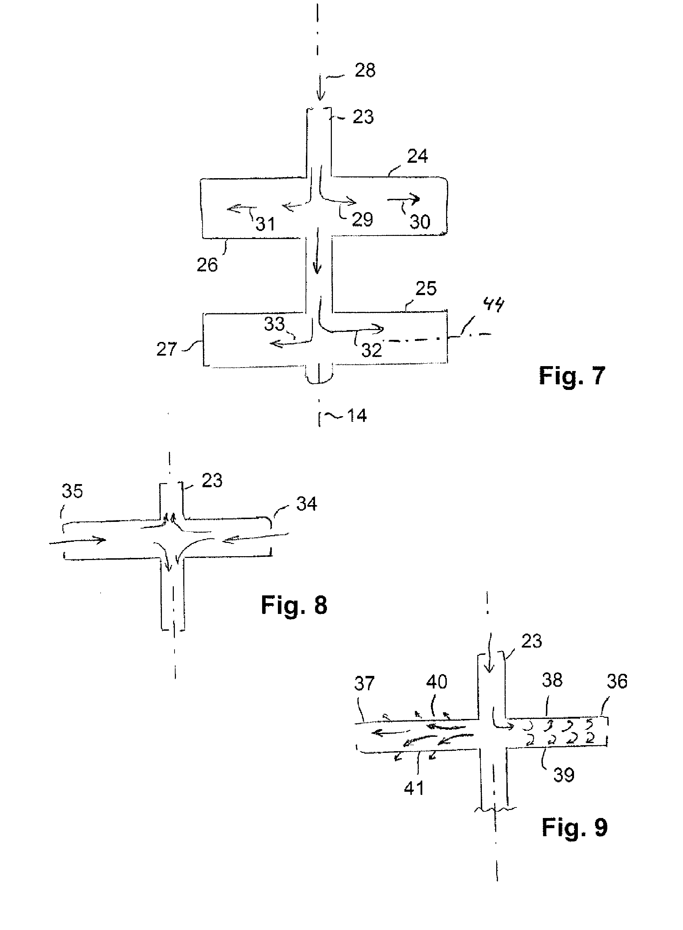

Anhand der Figur 7 soll nun auf die erfindungsgemäße Gestaltung eines Gusswerkzeugs / einer Gießform für einen erfindungsgemäßen Rotor eingegangen werden.

Es ist in Figur 7 ein Längsschnitt der Spritzgießform gezeigt, wobei der Bereich, der das Volumen der Rotornabe umfasst, mit 23 bezeichnet ist und die Volumina der einzelnen Förderelemente mit 24, 25, 26, 27 bezeichnet sind. In Figur 7 ist zudem eine Einspritzrichtung durch den Pfeil 28 angedeutet. Durch die weiteren Pfeile 29, 30, 31, 32, 33 ist angedeutet, dass der Spritzgusswerkstoff axial entlang der Rotationsachse 14 des entstehenden Rotors einströmt, und von dort aus radial nach außen in die Volumina der Förderelemente hinein. Beispielhaft ist die Längsachse eines der Förderelemente mit einer strichpunktierten Linie angedeutet und mit 44 bezeichnet. Werden in den Spritzgusswerkstoff entsprechend lange Fasern in ausreichender Anzahl eingebracht, so orientieren diese sich nach der Hauptströmungsrichtung des Werkstoffs und verbleiben beim Erstarren des Materials an Ort und Stelle.

Verdrängte Luft und überschüssiges Gießmaterial können am radial äußeren Ende der Förderelemente durch Öffnungen 45 abströmen.

In Figur 8 ist eine umgekehrte Einspritzrichtung angedeutet, wobei der Spritzgusswerkstoff von den radial äußeren Enden 34, 35 der Förderelemente radial nach innen zum Volumen des Rotors 23 eingespritzt wird. Auch in diesem Fall können lange Fasern mit eingebracht werden, die sich in der gemäß der Erfindung beabsichtigten Art und Weise orientieren und anordnen.

Anhand der Figur 9 wird gezeigt, dass bei schmalen Förderelementen 36, 37 die radial von innen nach außen strömende Vergussmasse an den Kanten 38, 39 des Volumens des jeweiligen Förderelements durch die Reibung verwirbelt, so dass sich keine laminare Strömung der Vergusswerkstoffs beim Einströmen ergibt.

Auf der linken Seite in der Darstellung der Figur 9 ist eine Gussformvariante dargestellt, die im Bereich der Kanten 40, 41 Überströmöffnungen oder Überströmschlitze aufweist, durch die ein Teil des Spritzgusswerkstoffs in Axialrichtung des Rotors abströmen kann, so dass die auf der rechten Seite der Figur 9 dargestellte Verwirbelung nicht entsteht. Im mittleren Bereich des Förderelements 37 ergibt sich damit eine quasi laminare Strömung, so dass die eingebrachten Fasern sich dort in gestreckter Form anordnen können, ohne dass sie durch Beeinflussung der Strömung des Spritzgusswerkstoffs verformt werden. Nach dem Erstarren können die Teile des Spritzgusswerkstoffs, die durch die Öffnungen an den Kanten 40,41 der Volumina der Förderelemente in der Spritzgießform ausgetreten sind, entfernt werden, beispielsweise durch Abschneiden. Gleiches kann, wie in Figur 7, an den Außenkanten der Förderelemente erfolgen. Hier sind zwei Überströmkanäle 45 vorgesehen, durch die der Kunststoff ohne Fasern entweichen kann.

Durch die oben angegebenen Merkmale, insbesondere durch Gestaltung des Rotors und Einbringen geeigneter Fasern, wird eine stabile Gestaltung eines Rotors mit ausreichender Formtreue auch nach teilweiser Überstreckung oder häufiger Wechselbelastung oder konstant anliegender Biegebelastung erreicht. Durch die erfindungsgemäßen Herstellungsverfahren und die dargestellte Spritzgießform wird eine sinnvolle und vorteilhafte Herstellungsmöglichkeit für den erfindungsgemäßen Rotor aufgezeigt.

## Patentansprüche

1. Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem komprimierten Zustand und einem expandierten Zustand komprimierbar und expandierbar ist und der wenigstens teilweise aus einem durch Fasern (10, 11, 13, 18, 19) verstärkten Kunststoff besteht und zur Rotation um eine Rotationsachse (14) vorgesehen ist, **dadurch gekennzeichnet, dass** der Kunststoff eine Shore-Härte < 100 D aufweist.

2. Rotor nach Anspruch 1, **dadurch gekennzeichnet, dass** ein erster Anteil von mehr als 30%, insbesondere mehr als 50%, der Fasern (10, 11, 13, 18, 19) im expandierten Zustand des Rotors (42) im Wesentlichen gestreckt von ihrem jeweils der Rotationsachse am nächsten liegenden Abschnitt (10a, 11a, 13a) zu einem der Rotationsachse ferner liegenden zweiten Abschnitt (10b, 11b, 13b) verläuft.

3. Rotor für eine komprimierbare Fluidpumpe, insbesondere eine durch ein Blutgefäß in einen Patientenkörper einführbare Blutpumpe, der ein oder mehrere Förderelemente (15) aufweist und radial zwischen einem komprimierten Zustand und einem expandierten Zustand komprimierbar und expandierbar ist und der wenigstens teilweise aus einem durch Fasern (10, 11,13, 18, 19) verstärkten Kunststoff besteht und zur Rotation um eine Rotationsachse (14) vorgesehen ist, **dadurch gekennzeichnet, dass** ein erster Anteil von mehr als 30%, insbesondere mehr als 50 %, der Fasern (10, 11,13, 18, 19) im expandierten Zustand des Rotors (42) im Wesentlichen gestreckt von ihrem jeweils der Rotationachse am nächsten liegenden ersten Abschnitt (10a, 11a, 13a) zu einem der Rotationsachse ferner liegenden zweiten Abschnitt (10b, 11b, 13b) verläuft, wobei der erste Anteil der Fasern eine Länge aufweist, die wenigstens 30 %, insbesondere 50 %, der Maximalhöhe der Förderelemente, gemessen in Radialrichtung des Rotors im expandierten Zustand, entspricht.

4. Rotor nach Anspruch 2 oder 3, **dadurch gekennzeichnet, dass** insbesondere im expandierten Zustand jede Faser (10, 11,13, 18, 19) des ersten Anteils der Fasern in ihrem Verlauf höchstens 45° in Axialrichtung und/oder Azimutalrichtung von einer radial auf die Rotorachse ausgerichteten Lage abweicht.

5. Rotor nach Anspruch 2 oder einem der folgenden, **dadurch gekennzeichnet, dass** insbesondere im expandierten Zustand jede Faser (10, 11 ,13, 18, 19) des ersten Anteils der Fasern im Wesentlichen senkrecht zur Rotationsachse verläuft.

6. Rotor nach Anspruch 2 oder einem der folgenden, **dadurch gekennzeichnet, dass** insbesondere im expandierten Zustand jede Faser des ersten Anteils der Fasern radial in Bezug auf die Rotationsachse (14) verläuft.

7. Rotor nach Anspruch 2 oder einem der folgenden, **dadurch gekennzeichnet, dass** jede Faser (10, 11, 13, 18, 19) des ersten Anteils des ersten Anteils der Fasern entlang der Längsachse (44) eines Förderelements verläuft.

8. Rotor nach Anspruch 2 oder einem der folgenden, **dadurch gekennzeichnet, dass** die Länge der Fasern (10, 11, 13, 18, 19) des ersten Anteils der Fasern wenigstens 10%, insbesondere wenigstens 30%, des Radius des Rotors beträgt.

9. Rotor nach Anspruch 1 oder einem der folgenden, **dadurch gekennzeichnet, dass** der Durchmesser der Fasern (10, 11, 13, 18, 19), insbesondere des ersten Anteils der Fasern, kleiner als 40 µm ist.

10. Rotor nach Anspruch 1 oder einem der folgenden, **dadurch gekennzeichnet, dass** die Förderelemente(15) aus einem Schaumstoff bestehen.

11. Verfahren zur Herstellung eines Rotors gemäß einem der Ansprüche 1 bis 10 mittels eines Gießverfahrens, insbesondere eines Spritzgießverfahrens, bei dem das Material der Förderelemente (15) in Radialrichtung in Bezug auf die Rotorachse (14) in die Volumina der Förderelemente derart eingebracht wird, dass der Spritzgusswerkstoff in die Volumina der Förderelemente jeweils in Radialrichtung (30, 31, 32, 33) hineinströmt.

12. Verfahren nach Anspruch 11, **dadurch gekennzeichnet, dass** der Spritzgusswerkstoff in die Volumina der Förderelemente (15) jeweils von dem der Rotorachse nächsten Bereich aus oder von dem der Rotorachse fernsten Bereich aus in radialer Richtung (30, 31, 32, 33) eingespritzt wird.

13. Gießform für einen Rotor gemäß einem der Ansprüche 1 bis 10, **dadurch gekennzeichnet, dass** bei den Volumina der Förderelemente(15) an deren radial verlaufenden Kanten (40, 41) Überströmkanäle vorgesehen sind, um einen störungsfreien Fluss des Gießwerkstoffs in Radialrichtung zu ermöglichen.
